Europäisches Patentamt

European Patent Office

Office européen des brevets

(11) Publication number: **0 070 738**
**B1**

# EUROPEAN PATENT SPECIFICATION

(45) Date of publication of patent specification: **14.06.89**

(51) Int. Cl.⁴: **A 61 M 1/34, B 01 D 13/00**

(21) Application number: **82303831.0**

(22) Date of filing: **21.07.82**

(54) **Plasmapheresis by reciprocatory pulsatile filtration.**

(30) Priority: **22.07.81 US 287116**
**16.02.82 US 349371**
**16.02.82 US 349367**

(43) Date of publication of application:
**26.01.83 Bulletin 83/04**

(45) Publication of the grant of the patent:
**14.06.89 Bulletin 89/24**

(84) Designated Contracting States:
**AT BE CH DE FR GB IT LI LU NL SE**

(56) References cited:
**DE-Á-2 925 143**
**GB-A-1 555 389**
**US-A-4 001 117**
**US-A-4 086 924**

(73) Proprietor: **E.I. DU PONT DE NEMOURS AND COMPANY**
**1007 Market Street**
**Wilmington Delaware 19898 (US)**

(72) Inventor: **Duggins, Ray Brown**
**McCarthy Drive, R.D.1**
**Chadds Ford Pennsylvania 19317 (US)**
Inventor: **Ramstack, Joseph Michael**
**946 North Hill Drive**
**West Chester Pennsylvania 19380 (US)**

(74) Representative: **Jones, Alan John et al**
**CARPMAELS & RANSFORD 43 Bloomsbury Square**
**London, WC1A 2RA (GB)**

Courier Press, Leamington Spa, England.

**Description**

TECHNICAL FIELD

This invention pertains to process and apparatus for plasmapheresis by reciprocatory pulsatile filtration with microporous membranes.

BACKGROUND INFORMATION

Plasmapheresis is a process of separating plasma from whole blood. The plasma-depleted blood is comprised principally of cellular components, e.g., red blood cells, white blood cells and platelets. Plasma is comprised largely of water, but also contains proteins and various other noncellular compounds, both organic and inorganic.

Continuous plasmapheresis is the process of continuously separating plasma from whole blood.

Plasmapheresis is currently used to obtain plasma for various transfusion needs, e.g., preparation of fresh-frozen plasma, for subsequent fractionation to obtain specific proteins such as serum albumin, to produce cell culture media, and for disease therapies involving either the replacement of plasma or removal of specific disease-contributing factors from the plasma.

Plasmapheresis can be carried out by centrifugation or by filtration. Generally, in known filtration apparatus, whole blood is conducted in a laminar flow path across one surface, i.e., the blood side surface, of a microporous membrane with a positive transmembrane pressure difference. Useful microporous membranes have pores which substantially retain the cellular components of blood but allow plasma to pass through. Such pores are referred to herein as cell-retaining pores. Typically, cell-retaining pore diameters are 0.1 μm to 1.0 μm.

In such known apparatus, as the blood flows through the flow path, the cellular components tend to migrate towards the center or axis of the path. Ideally, plasma occupies the periphery of the path so that it is predominantly plasma that contacts the membrane. A pressure difference across the membrane causes some of the plasma to pass through the pores of the membrane while plasma-depleted blood continues to flow to the end of the path. Ideally, the filtrate is cell free; the plasma-depleted blood collected at the end of the flow path is concentrated, i.e., is depleted in plasma and therefore has an increased hematocrit (volume percent of red blood cells).

After blood has been conducted across the surface of a membrane at normal venous flow rates for some time, the transmembrane flow of plasma becomes impaired. This phenomenon is herein sometimes referred to as membrane fouling or simply as fouling. Known techniques for reducing fouling, i.e., increasing the length of time for which the process can be caried out without the occurrence of significant impairment of plasma flow, include varying the flow path size so as to optimize the wall shear rate along the length of the flow path as disclosed in U.S. Patent 4,212,742, and recycling a portion of the plasma-depleted blood to increase the velocity of blood in the flow path; the latter technique may result in less plasma-depletion.

Various filtration devices for plasmapheresis are disclosed in the literature. U.S. 3,705,100 discloses a center-fed circular membrane having a spiral flow path. U.S. 4,212,742 discloses a device having divergent flow channels. German Patent 2,925,143 discloses a filtration apparatus having parallel blood flow paths on one side of a membrane and parallel plasma flow paths, which are perpendicular to the blood flow paths, on the opposite surface of the membrane. U.K. Patent Application 2,037,614 discloses a rectilinear double-membrane envelope in which the membranes are sealed together at the ends of the blood flow path. U.K. Patent Specification 1,555,389 discloses a circular, center-fed, double-membrane envelope in which the membranes are sealed around their peripheries. German Patent 2,653,875 discloses a circular, center-fed double-membrane device in which blood flows through slot-shaped filter chambers.

US—A—4 001 117 discloses apparatus for sieve filtration, and in particular ultrafiltration using a membrane as the filter, said to be particularly useful for filtering extremely small initial volumes, in particular of blood plasma or serum, in a pressurized cell with alternating flow direction of the liquid to be filtered, thus preventing concentration polarization, and preventing contact of the liquid to be filtered with any foreign substance such as a pressure gas. This reference does not disclose reciprocatory pulsatile flow.

It is an object of this invention to provide process and apparatus for plasmapheresis by filtration. It is a further object to provide such process and apparatus whereby highly concentrated, plasma-depleted blood can be continuously collected without significant hemolysis and with reduced membrane fouling. Another object is to provide process and apparatus for imparting reciprocatory pulsations to blood in a blood flow path during plasmapheresis by filtration.

BRIEF DESCRIPTION OF THE DRAWINGS

FIG. 1 is a cross-section of a double-membrane filtration module which may be used in the process of the invention, taken along line I—I of FIG. 2.

FIG. 2 is a perspective view of an illustrative embodiment of the filtration module of FIG. 1 having a loop and an oscillator to oscillate blood in a blood flow path between inlet and outlet.

FIG. 3 is a perspective view of a module having an end plate which has reciprocatory pulse cavities.

FIG. 4 is an exploded view of a module which is preferred for use in the invention.

FIG. 5 is a plan view of a blood side support of the module of FIG. 4.

FIG. 6 is a plan view of a plasma side support of the module of FIG. 4.

FIG. 7 is a cross-sectional view of a central sealing region in the module of FIG. 4.

FIG. 8 is an elevational view in cross-section of the attachment of an inlet or outlet tube to the module of FIG. 4.

FIG. 9 is a cross-sectional view of a module of the invention pressed between clamp jaws.

FIG. 10 is an elevational view in cross-section of a pumping arrangement used with the module of FIG. 4.

FIG. 11 is a perspective view of an alternative blood side support which may be used in the invention.

FIG. 12 is a perspective view of an alternative plasma side support which may be used with the blood side support of FIG. 11.

FIG. 13 is a plan view of a second illustrative embodiment of a module which is useful in the invention and can be used with the supports of FIGS. 11 and 12.

DISCLOSURE OF THE INVENTION

For further comprehension of the invention and of the objects and advantages thereof, reference may be made to the following description and to the appended claims in which various novel features of the invention are more particularly set forth.

It has been found that the above objects can be achieved by conducting blood over the surface of a membrane in reciprocatory pulsatile flow. In particular, the invention resides in a method for separating plasma from blood, which method comprises:

(1) conducting blood in a forward direction over a first surface, i.e., a blood side surface, of each of one or more membranes having cell-retaining pores, while maintaining a net positive transmembrane pressure difference;

(2) terminating the forward conducting of blood over the first surface of the membrane;

(3) conducting the blood in the reverse direction over said first surface, the volume of blood flowed in the reverse direction being less than the volume of blood flowed in the forward direction in step (1);

(4) repeating steps (1)—(3) in sequence to superimpose oscillatory pulsatile flow on the continuous forward flow of the blood, and collecting plasma which passes through each membrane from a second surface, i.e., a plasma side surface thereof and collecting plasma-depleted blood from said first surface, with the proviso that said plasma and said plasma-depleted blood are not returned to the donor body from which the blood is drawn.

The invention further resides in said process wherein the transmembrane pressure difference is reduced between periods of forward flow, preferably to below zero.

The invention also resides in apparatus for carrying out the aforesaid steps. In particular, the invention also resides in apparatus for continuously separating plasma from blood, which apparatus comprises one or more membranes having cell-retaining pores, means for conducting blood forward at a net positive transmembrane pressure difference and reverse over a first surface of each membrane whereby to superimpose oscillatory pulsatile flow on the continuous forward flow of the blood, means for collecting plasma which passes through each membrane from a second surface, i.e., a plasma side surface, thereof and means for collecting plasma-depleted blood from said first surfae. The invention also resides in said apparatus comprising means for reducing the transmembrane pressure difference between periods of forward flow, preferably, means for reducing said pressure difference to below zero.

Further, the invention resides in the membrane filter module which comprises:

first and second opposing module housing plates having circular recesses within opposing surfaces so as to form a blood flow region between two plasma flow regions, there being a central blood inlet port connected to the blood flow region; a blood collection channel, around the blood flow region, connected to a plasma-depleted blood outlet port; and a plasma collection channel around each plasma flow region connected to a plasma outlet port;

a plasma-side support within each plasma flow region; and

a pair of membranes, having cell-retaining pores, between each plasma flow region and the blood flow region, there being an elastomeric seal between each membrane and each plate and a blood flow path between the membranes.

The invention also resides in such a filtration module in which blood side supports are located between the membranes. Such module may have means for imparting reciprocatory pulsatile flow to blood in the flow path connected thereto.

In addition, the invention resides in a method for imparting reciprocatory pulsations to blood in a blood flow path on a surface of a membrane which comprises oscillating the blood between an inlet and an outlet. The invention also resides in said method which comprises superimposing oscillatory flow on a continuous forward flow of the blood and in the particular method which comprises oscillating the blood in a circuit which includes the blood flow path and a loop extending between an inlet and an outlet of the flow path.

The invention also resides in an improved plasmapheresis filtration module having a blood flow path on a surface of a membrane wherein the improvement comprises means for oscillating blood between an inlet and an outlet of the flow path, and also means for superimposing oscillatory flow on a continuous forward flow of the blood. A particular apparatus includes a circuit which includes the flow path and a loop extending between an inlet and an outlet of the flow path, the loop being provided with oscillating means. The module may be of any suitable shape or configuration.

In general, it has been found that plasmapheresis by filtration is enhanced by the use of fouling-reducing techniques, e.g., pulsatile flow, reciprocatory pulsatile flow and high blood flow rate via recirculation. Other steps may also be included, e.g., recycling the plasma-depleted blood, treating plasma during filtration, diluting the blood with a compatible fluid and measuring various biologically significant factors.

By comprises is meant that the invention includes the aforesaid steps and elements although it is to be understood that other steps and elements are not excluded from the invention, e.g., recycling the plasma-depleted blood, treating plasma during filtration, diluting the blood with a compatible fluid and measuring various biologically significant factors and means therefor.

In the following description and examples of the invention, the term "forward" is used to indicate a direction generally away from the source of blood; reverse indicates a direction generally towards the source of blood. Transmembrane pressure difference is determined by subtracting the pressure on the plasma side, i.e., the second surface of the membrane, from the pressure on the blood side, i.e., the first surface of the membrane. The term "fouling" is used to describe the impairment of plasma flow through a membrane. "Cell-retaining pores" means pores which substantially retain cellular components but allow plasma to pass through the membrane.

In the invention, blood may be conducted in a forward direction in a flow path over the first surface of a membrane by any means which does not cause significant damage to cellular components, which does not cause significant discomfort or danger to a donor or patient, which provides sufficient forward flow rate and pressure to efficiently fractionate blood in the manner and under the conditions described below, and which allows the forward flow to be periodically interrupted as described below. Examples include various pumps such as a rotary peristaltic pump, a piston or syringe pump, and a plunger or hose pump; even manually operated devices such as a flexible blood-containing chamber which can conduct blood forward when compressed may be used.

The membrane is made of any blood-compatible material, and has cell-retaining pores, i.e., pores which substantially retain cellular components but allow plasma to pass through; such pores are typically about 0.1 to 1.0 μm average diameter. The selection of a pore size may vary with the goal of a particular treatment. Useful membranes are described in some of the above-cited references relating to plasmapheresis. The membrane may be of any suitable shape, e.g., tubular, such as hollow fibers or any planar shape. When planar membranes are used, membranes having low elongation, e.g., less than about 65%, high modulus, e.g., at least about 10 kpsi (70 MPa), and high tensile strength, e.g., at least about 3000 psi (20 MPa), when tested wet in accordance with standard procedures, are preferred, because they are dimensionally stable. As exemplary of membranes having these preferred properties are mentioned the HT 450 polysulfone membrane commercially available from Gelman Sciences, Inc. and the polyester and polycarbonate membranes commercially available from Nuclepore Corporation. Of these, thin, e.g., less than about 1 mil (25 μm), preferably less than 0.5 mil (13 μm), smooth polycarbonate or polyester capillary pore membranes are preferred because, in laboratory experiments, such membranes were found, in general, to perform better than the tortuous path membranes which were tested. Under various conditions of practice, however, any of the above-described or other types of membranes may prove to be more or less advantageous. It is to be understood that more than one membrane in any arrangement may be used. Conveniently, several membranes are stacked within an enclosed module so that blood is fractionated by more than one membrane simultaneously. A planar membrane is preferably supported on the plasma side and more preferably on both sides by, e.g., supports comprising plates having grooves, pores or projections or fabric-like materials. A preferred plasma side support comprises a plurality of layers of a nonwoven polyester fabric.

From the location at which the blood first contacts the membrane, which may or may not be near a point on an edge or end of the membrane, blood is conducted in a forward direction in one or more flow paths. A flow path is the space through which the blood flows on the first surface of the membrane. For example, in a preferred embodiment, the membrane is planar and circular, the location at which the blood contacts the membrane is near the center thereof, and the flow path extends radially, ending near the periphery of the membrane. It is apparent that when the membrane is tubular and blood is conducted within the tube, the membrane may alone define the flow path. Typically the depth of blood in each flow path is less than about 30 mils (0.76 mm). Preferably, said depth is also at least about 4 mils (0.10 mm) but, preferably no more than about 10 mils (0.25 mm).

The rate at which blood is conducted over the first surface of the membrane is at least as high as may be needed to provide a net positive transmembrane pressure difference. The flow velocity typically varies during each period of forward flow. The preferred average forward flow rate from the source to the membrane is about 50 to 60 mL·min$^{-1}$ when the source of blood is a vein of a normal human donor although the process may be carried out at higher or lower flow rates.

Plasma is driven through the cell-retaining pores in the membrane at a practical rate by a positive transmembrane pressure difference. Typically, positive transmembrane pressure difference is generated primarily by resistance to forward flow, but it can also be generated in other ways, e.g., by decreasing pressure on the plasma on the second surface.

It has been found that the amount of transmembrane pressure difference that can be withstood by blood without hemolysis is largely a function of cell-retaining pore size. For most purposes, the preferred pore diameter is about 0.4 to 0.6 µm. In this range, a positive transmembrane pressure difference of up to about 4 psi (28 kPa) is desirable although up to about 1.5 psi (10 kPa) is believed to be preferred. When the pore diameter is smaller or larger, higher or lower transmembrane pressure differences, respectively, are acceptable. It is to be understood that the pressure on the blood side and the plasma side surfaces, and the transmembrane pressure difference, may vary during the course of a treatment and in different regions of the flow path.

After the forward conducting of blood over the first surface of the membrane with a positive transmembrane pressure difference is continued for some tiome, the membrane becomes progressively fouled, i.e., the flow of plasma through the membrane becomes increasingly impaired. The length of time for which blood can be so conducted is believed to depend upon several factors such as, e.g., flow velocity, hematocrit, pore size, transmembrane pressure difference, and the individual characteristics of the blood being treated. The frequency and volume of the reciprocatory pulses are selected to maximize the flow of plasma through the membrane without causing extensive blood trauma. In planar blood flow paths having a height of about 4 to 10 mils (100 to 254 µm), a useful frequency and volume are about 20 to 140 pulsations per minute and 0.5 to 4 mL per pulsation, preferably about 3 mL. Said parameters should be selected to provide a mean linear velocity up to about 400 mm-sec$^{-1}$, preferably, up to about 250 mm-sec$^{-1}$. These parameters may be adjusted during a particular treatment, but conveniently may be selected and fixed for an entire treatment.

After the forward conducting of blood is terminated, blood is conducted in the reverse direction in each flow path. The termination of forward flow and the conducting of blood in the reverse direction need not occur simultaneously over the entire membrane. Because blood is conducted in forward and reverse directions with a net forward flow during the procedure, the blood flow is referred to as reciprocatory pulsatile flow.

In a preferred embodiment, the transmembrane pressure difference is reduced when the conducting of blood in the forward direction is substantially terminated, i.e., between periods of forward flow. Typically, said reduction is accomplished as a result of the changes in the direciton of blood flow although other means, such as means for increasing plasma-side pressure, may also be useful. It is to be understood that said reduction need not occur simultaneously over the entire membrane, e.g., at any given instant, there may be areas on the membrane with high transmembrane pressure difference and other areas with low transmembrane pressure difference and, at any given point on the membrane, the transmembrane pressure difference may continuously fluctuate. Preferably, the transmembrane pressure difference is reduced to below zero, e.g., about −.1 to −3.0 psi (−.7 to −20.7 kPa), and, more preferably, to about −0.8 to −1.0 psi (−5.3 to −6.9 kPa). Preferably, a large amount of plasma backflow through the membrane is avoided.

The duration of the reverse flow of blood is selected to maintain substantially unimpaired flow of plasma through the membrane as well as to increase the distance which the blood travels across the membrane. A wide range of reverse flow durations are useful. The volume of blood flowed in the reverse direction is less than the volume of blood flowed forward.

It is to be understood that reverse flows of blood may begin in some regions of the flow paths prior to cessation of the forward flow of blood in other, or even in the same, regions, i.e., forward and reverse flows may overlap. It is preferred that the frequency of the reciprocatory pulsations be low in the early stages of a treatment and then be gradually raised to a desirable frequency. It may be necessary to adjust the apparatus during a procedure to maintain desirable pressures and flows.

The blood which approaches the ends of each flow path is plasma-depleted blood. It is collected and conducted away from the membrane by any suitable means, as is the plasma which flows through the membrane.

The reciprocatory pulsations and transmembrane pressure difference reductions, as is apparent from the above discussion, can be carried out in numerous ways. Typically, the means include a plurality of coordinated pumps and valves positioned on blood, plasma-depleted blood and/or plasma lines. Pressure accumulators, or surge chambers, may also be useful. Some such useful means are disclosed in the following examples, which are illustrative only, of treatments in accordance with the invention. Other means will be obvious to persons skilled in the art.

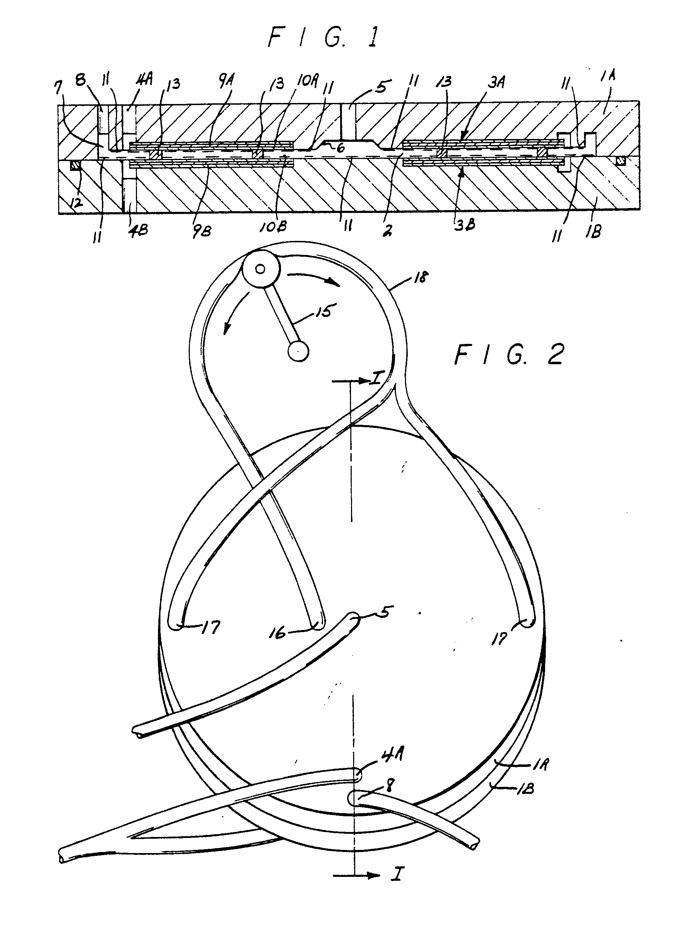

Referring to FIG. 1, a filtration module, which may be used with reciprocatory pulsatile flow and may have means for generating reciprocatory pulsations connected thereto, comprises two circular opposing module housing plates 1A, 1B which are prepared from a blood-compatible material. A circular blood flow region 2 is recessed within an opposing surface of one or both plates. Further recessed within each plate is a plasma flow region 3A, 3B. Typically, though not necessarily, the plasma flow region is of smaller diameter than the blood flow region.

The depth of the plasma flow region is typically about 5 to 20 mils (127 to 508 µm). The surface of the plasma flow region may be smooth or grooves to enhance radial flow of plasma. In the plasma flow region, or connected thereto, may be means for treating the plasma for the removal of disease-contributing factors.

One or both plates 1A, 1B have plasma outlet ports 4A, 4B connected to the plasma flow regions 3A, 3B via a plasma collection channel around the plasma flow regions, e.g., about 3 mm deep and 1.5 mm wide. There may be one or more of such ports in either or both plates. The ports and channel may be located at any position but preferably, as herein illustrated, are located near the periphery of each plasma flow region.

Near the center of plate 1A is blood inlet port 5, the walls 6 of which extend through plasma flow region 3A to the blood flow region 2. Around the periphery of blood flow region 2 is a plasma-depleted blood collection channel 7. This channel connects to one or more plasma-depleted blood outlet ports 8.

Within each plasma flow region is a plasma side membrane support 9A, 9B which may be, e.g., a plate having grooves, pores or projections or fabric-like materials. As illustrated, the plasma side supports are comprised of layers of fabric-like materials, such as layers of a nonwoven polyester fabric. The preferred support is three layers of 4 mil (102 µm) thick Hollytex, made by calendering Du Pont Reemay® spunbonded polyester, because it provides adequate support while allowing transverse and radial flow of plasma. The support 9A which fits in plasma flow region 3A is provided with an aperture which fits around wall 6 of blood inlet port 5.

Within each blood flow region is a membrane 10A, 10B. Membrane 10A which fits in blood flow region 2 in plate 1A is provided with an aperture which lies approximately in registry with blood inlet port 5.

The membranes 10A, 10B are adhered to the plates near the peripheral edges of the membranes and, in the case of the membrane 10A, near the edge of the aperture in the membrane which is in registry with blood inlet port 5, with an elastomeric adhesive. Use of an elastomeric seal provides sufficient flexibility to avoid rupture of the membranes during use. The areas of membranes 10A, 10B which are adhered to plates 1A, 1B are identified in FIG. 1 by the number 11.

It has been found that when thin polycarbonate or polyester membranes which have low break elongation, i.e., less than about 40%, are employed in filter modules in which, as herein illustrated, the membranes are not rigidly supported across a large part of their surface areas, it is advantageous to employ an elastomeric seal between the membranes and supports. Use of an elastomeric seal provides sufficient flexibility to avoid rupture of the membranes during use. When such membranes are employed, the seal preferably has a break elongation of at least about 100%. The optimal break elongation will depend on several factors which will be obvious to persons skilled in the art, including the thickness of the seal. An elastomeric seal which has been found to perform well with such membranes is an adhesive having a break elongation of about 400% and applied in a layer about 3 mils (76 µm) thick.

When the module is assembled, the corresponding flow regions of each plate are adjacent. The plates are held together by any suitable means, e.g., clamps, bolts and adhesives. An O-ring 12 can be used to seal the plates. The region between the membranes is the blood flow path. The total effective surface area of the membranes, i.e., the sum of the areas on both membranes through which plasma can flow, is about .02 to .06 m².

Blood side supports 13 are located between the membranes. Blood side supports, though not necessary, have been found to be advantageous when nonrigid plasma side supports, such as layers of Hollytex, which may tend to buckle during use, are employed. Various suitable supports are described in the literature. The illustrated and preferred supports comprise a plurality of smooth pillars, e.g., substantially circular, dots of a material which has sufficient softness to avoid breakage of the membranes during use, such as an elastomeric adhesive. Conveniently, if an adhesive is used to adhere the membranes to the plates, the same adhesive is used to form the blood side supports.

FIG. 2 is an illustration of an embodiment of the invention in which the filtration module of FIG. 1 is used. Blood is conducted from the source to the blood flow path via blood inlet port 5 in module housing plate 1A. Plasma which passes through the membranes exits from the module through a plasma outlet port 4A, and a second plasma outlet port, not shown. Plasma-depleted blood from the end of the blood flow path exits from the module through plasma-depleted blood outlet port 8. In addition, blood flow is pulsed in reciprocatory fashion by a peristaltic oscillator 15, which is connected to central and peripheral ports 16 and 17 through loop 18, which peripheral ports are connected to areas near an end of the flow path, directly, or indirectly via a blood collection channel, not shown. The loop is preferably short so that blood in the loop is frequently mixed and exchanged with blood in the flow path. There preferably is little or no exchange of blood across the oscillator. Any suitable type of pump may be used to cause the reciprocatory pulsations. Such pumps are described in the literature and in the Examples below; a peristaltic pump is preferred. Preferably, though not necessarily, the oscillator is connected to the blood flow path via one centrally located port and two peripherally located ports, as shown, or to the blood inlet and plasma-depleted blood outlet lines at a location close ot the module. The duration and frequency of oscillations can be regulated by adjusting the oscillator. The forward and reverse strokes are typically of equal volume.

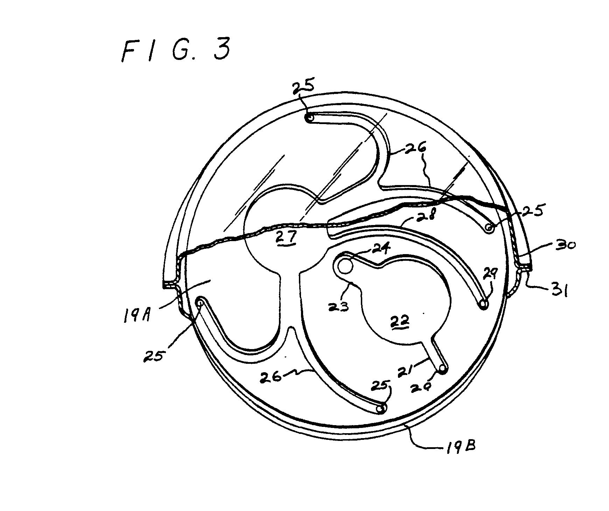

FIG. 3 illustrates a module having an end plate, i.e., module housing plate, which has recoprocatory pulse cavities integral therewith. The end plate is not the invention claimed herein but is included as illustrative of an embodiment which is useful with the invention herein.

Blood is conducted into the module via an inlet, not shown, in end plate 19B and is conducted through a matched port 20 in end plate 19A. From port 20 in end plate 19A, the blood is conducted through shallow channel 21, 0.2 inch (5.1 mm) wide × 0.06 inch (1.5 mm) deep, into inlet reciprocatory pulse cavity 22 which has a volume of about 3 mL and is about 2 inches (50.8 mm) in diameter × 0.06 inch (1.5 mm) deep. Cavity 22 is employed in the generation of reciprocatory pulsations as described below. From cavity 22, the blood is conducted through shallow channel 23, 0.5 inch (127 mm) wide × 0.13 inch (3.3 mm) deep, to blood flow path inlet 24 which is about 0.38 inch (9.7 mm) in diameter. The blood is conducted through port 24 into a blood flow region between two membranes as described above. Plasma-depleted blood is conducted through flow path outlets 25 and through branch channels 26 to outlet reciprocatory pulse cavity 27 in end plate 19A. The branch channels from the four outlets 25, which are equidistant from each other, begin as four channels each about .250 inch (6.4 mm) wide × .060 inch (1.5 mm) deep and merge into two channels each about .500 inch (12.7 mm) wide × .060 inch (1.5 mm) deep. The branch channels are of equal length and cross-section so as to produce substantially equal pressure conditions during use. Cavity 27 is also employed in the geenration of reciprocatory pulsations as described below. From cavity 27, the plasma-depleted blood is conducted through shallow channel 28, .200 inch (5.1 mm) wide × .060 inch (1.5 mm) deep, and through plasma depleted blood outlet 29 which extends through a matched port in end plate 19B, i.e., cavity 27 is between blood flow path outlets 25 and module plasma-depleted blood outlet 29.

Plasma which passes through the membranes flows radially in a plasma flow path and through a plasma collection channel, as described above, to an outlet port, not shown, in end plate 19B.

The entire module is enclosed by envelope 30 which is comprised of two sheets of a flexible blood impermeable material, such as poly(vinyl chloride), the sheets being joined together at seal 31 around the perimeter of the stack. The envelope thus provides a unitary flexible enclosure for the module. The three apertures in end plate 19B mate with tube connectors in envelope 30.

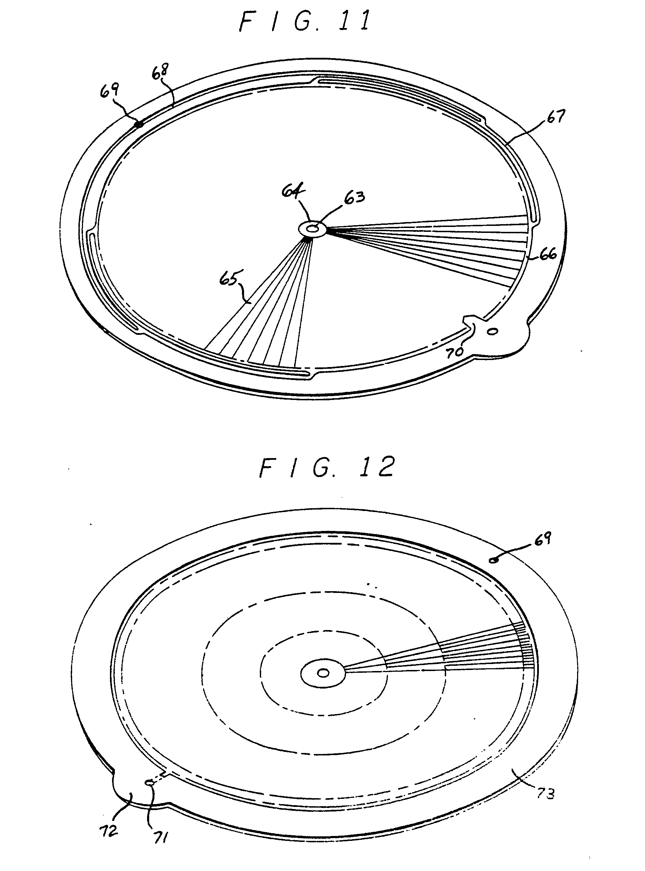

Envelope 30 covers and seals the various channels, cavities and apertures in end plate 19A and forms a flexible diaphragm over each cavity 22, 27. A perimeter lip, not shown, around each cavity and channel in end plate 19A aids in sealing. Reciprocatory pulsations are generated by alternately compressing the diaphragm over each cavity 22, 27 such as by the use of reciprocating plungers. Reciprocating plungers which are useful for this purpose are illustrated in FIG. 10.

All of the above illustrated modules must be clamped using pressure which is at least sufficient to offset internal pressure. In the examples, below, a series of C-clamps around the perimeter of each module was employed.

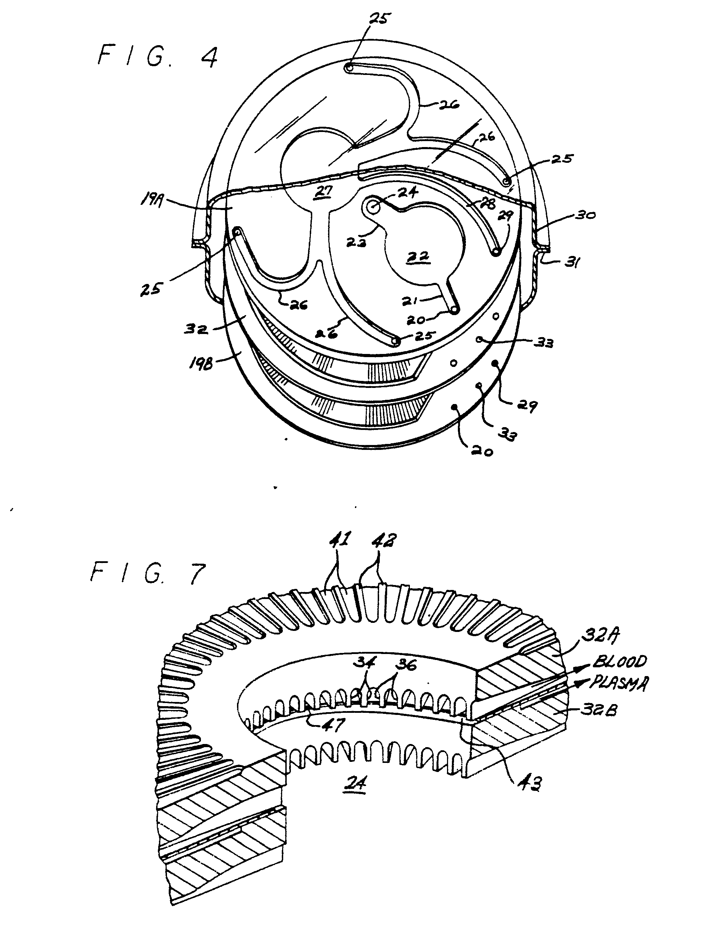

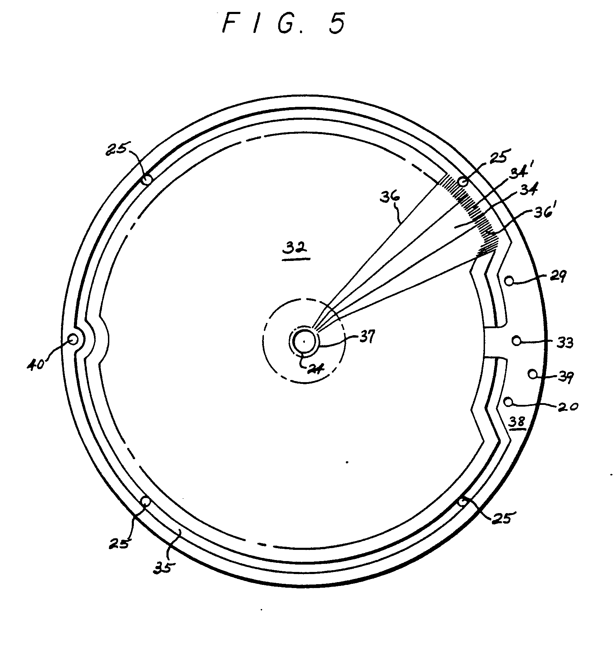

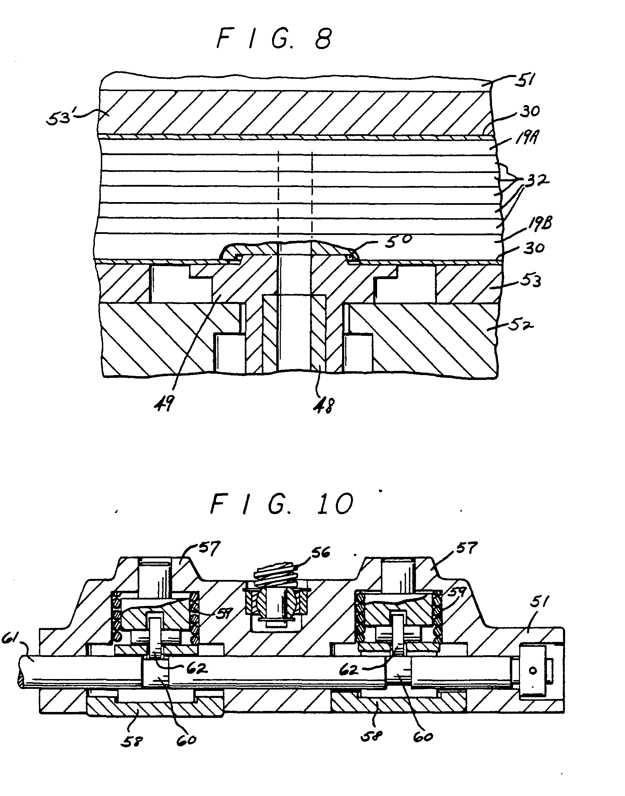

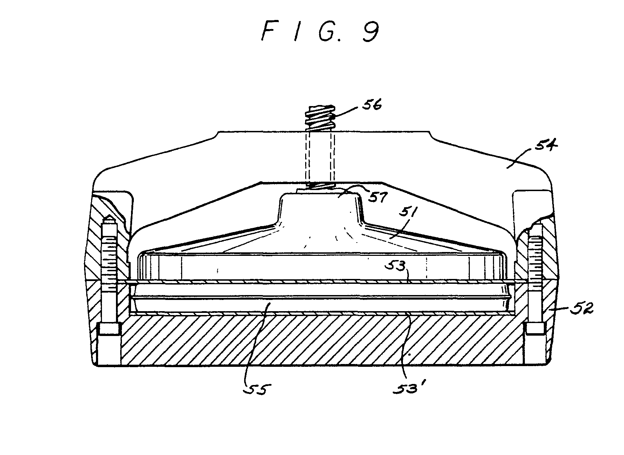

FIG. 4 is illustrative of a two membrane filter module having an end plate as described above in FIG. 3. The module and end plate are not the inventions claimed herein but are included because they are illustrative of a means for carrying out this invention and of the preferred module comprising said means. FIGS. 5 to 9 illustrate particular elements of the module of FIG. 4. Referring to FIG. 4, the module comprises a clampable stack of plates 19A, 19B, 32, between which, suitable membranes, not shown, are interleaved. The plates are flexible and require external structural support, such as is described below with reference to FIGS. 8 and 9, to effect sealing and to compensate for compliance and tolerance within the module. Blood is conducted into the module via inlet 20 in end plate 19B and is conducted through matched ports in plates 32, 19A. End plate 19A is about 0.19 inch (4.8 mm) thick; end plate 19B and plate 32 are about 0.08 inch (2.0 mm) thick; the module is about 8 inches (0.2 μm) in diameter.

From module inlet 20 in end plate 19A, the blood is conducted to port 24, as described above in FIG. 3, and through matched ports in plates 32 and in the membranes, to blood flow paths lying between each membrane and one surface of a plate, e.g., on a membrane lying between end plate 19A and adjacent plate 32, the blood flow path is between the membrane and the interior surface of end plate 19A, which is a blood side support, as illustrated for plate 32 in FIG. 5. The blood in the blood flow paths is conducted radially to plasma-depleted blood collection channels and from there, through matched flow path outlets 25 as described in FIG. 3.

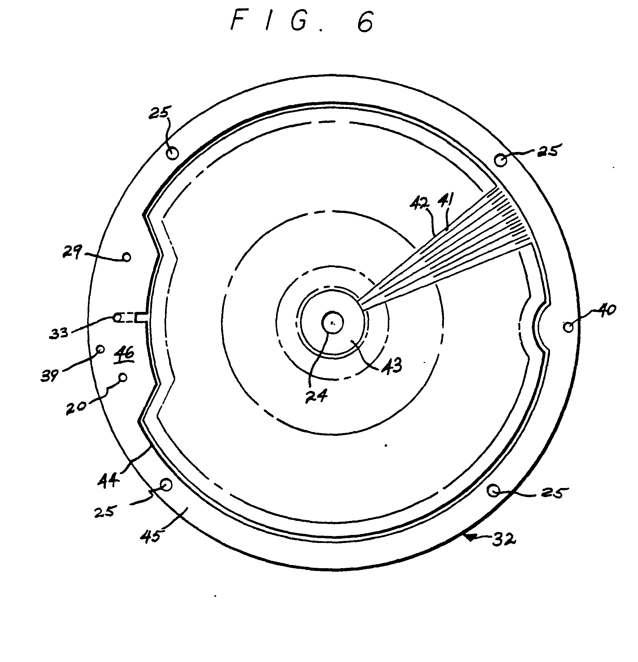

Plasma which passes through the membranes flows radially in a plasma flow path, e.g., on the membrane which lies between end plate 19A and adjacent plate 32, the plasma flow path is between the membrane and plate 32. The plasma flow path is comprised of radial flow channels which culminate in a perimeter plasma collection channel, as illustrated by FIG. 6, from which the plasma passes through matched ports 33 in plates 32, 19B and out of the module. A section of plasma flow channels is illustrated in FIG. 4. The entire module is enclosed by envelope 30 as described above in FIG. 3.

FIG. 5 illustrates a blood side support comprised of plate 32, a surface of which is provided with recessed radial blood flow channels 34. Between channels 34 are ridges 36. The channels 34 extend from counterbore 37 around inlet 24. For purposes of illustration, only a portion of enlarged blood flow channels are shown. In fact, ninety channels 34 extend around the entire perimeter of inlet 24 although more or fewer channels may be employed. The channels 34 are at least about 4 mils (.1 mm) deep, preferably about 4 to 10 mils (.1 to .3 mm). They are narrow around the inlet and increase in width from about 8 mils (.2 mm) to about 250 mils (6.4 mm). The counterbore is about 20 mils (.5 mm) deep and .5 inch (12.7 mm) in diameter. Around the perimeter of flow channels 34 is perimeter plasma-depleted blood collection channel 35 which leads to plasma-depleted blood outlet ports 25. Between flow channels 34 and collection channel 35 are blood pressure balancing and sealing grooves comprising a perimeter border of short narrow channels 34', each about 4 to 30 mils (.1 to .8 mm) wide. Between perimeter channels 34' are ridged 36'. Perimeter channels 34' enhance uniform distribution of pressure and flow within the blood flow channels by causing increased velocity and hence increased pressure drop across the perimeter channels.

In region 38, the channels are spaced inward from the edge of the plate so as to avoid intersecting any of ports 20, 33, 29. The channels 34 are offset from radial plasma flow channels on a plasma side support so that the ridges between the blood flow channels and the ridges between the plasma flow channels will not be contiguous but rather will intersect, thus minimizing the risk of membrane shearing; in the illustrated embodiment, approximately the outer 80% of the axes of flow channels 34 are angled slightly from a pure radial direction. Also to minimize the risk of shearing, the ridges between the channels preferably have flat surfaces, e.g., about 3 to 10 mils (.1 to .3 mm) wide.

Alignment pins 39 and 40 fit snugly into aligned holes in each plate 19A, 19B, 32, thus maintaining the plates in the proper relative orientation.

The preferred plasma side support, opposite the blood side support, is illustrated by FIG. 6. The plasma side support comprises the other surface of plate 32, having plasma flow channels 41 recessed in one surface thereof with ridges 42 therebetween. The plasma flow channels 41 extend from a central sealing surface 43 in zones of progressively greater numbers to a perimeter plasma collection channel 44, which is about 0.07 inch (1.8 mm) wide × 0.030 inch (.8 mm) deep. For purposes of illustration, only a section of enlarged plasma flow channels are shown. By progressively increasing the numbers of plasma flow channels, closely-spaced ridges, which provide support for the membrane, are maintained. In the illustrated plasma side support, the number of plasma flow channels doubles in each succeeding zone so that in the innermost zone there are 90 such channels and in the outermost zone there are 1440 such channels.

In the center of plate 32 is a blood flow path inlet 24, e.g, about .39 inch (9.9 mm) in diameter, which is in registry with blood flow path inlet 24 in plate 19A.

The inlet sealing surface 43 is an area on the plasma side support which is coplanar with the nonrecessed areas of the support. It is opposite narrow blood flow channels on an opposing blood side support so that when the supports are pressed together with a membrane therebetween, blood is substantially prevented from leaking into plasma flow regions without the use of adhesives or gaskets. Surface 43 is a circular area, concentric with inlet 24 and of larger diameter, e.g., about 1 inch (25.4 mm). Preferably, it is an inlet sealing boss although other elements can be used, e.g., an annular insert. It substantially prevents blood from leaking from inlet 24 to plasma flow channels 41. The plasma collection channel 44 is located within a smaller radius than the short, narrow channels 34' on plate 19A. Between the plasma collection channel 44 and the edge of plate 32 is a perimeter sealing surface 45 which can be pressed against channels 34', there being a membrane therebetween, effecting a seal in a manner similar to the seal around inlet 24.

From the plasma collection channel 44, plasma flows to plasma outlet 33. As with the blood side support, the channels are spaced inward from the edge of the plate in region 46.

The interior surface of plate 19A in FIG. 4 also comprises a blood side support identical to that shown in FIG. 5. Several plates 32 can be stacked to permit use of a desired number of membranes, the preferred number being four to six. The last plate, i.e., end plate 19B, comprises a plasma side support, on its interior surface, which is identical to the plasma side support illustrated in FIG. 6 except that end plate 19B is not apertured with blood flow path inlet 24. On its exterior surface, end plate 19B is plain.

FIG. 7 illustrates the central sealing design of the module and the preferred blood flow path entrance design. The membrane 47 is pressed between the blood side support surface of one plate 32A and the plasma side support surface of a second plate 32B. In this figure, there is no counterbore around the inlet as there is in FIG. 5. Membrane 47 bridges the narrow blood flow channels around inlet 24 and is squeezed against central sealing boss 43 of the next plate, acting in this region as seal members in a manner similar to a check valve. By employing channels which are about 4 to 20 mils (.1 to .5 mm), preferably 6 to 10 mils (.2 to .3 mm), in width, under usual operating conditions, i.e., pressures up to about 3 psi (21 kPa), the membrane seal has been found to substantially prevent leakage of blood even when reciprocatory pulsatility is employed, when the module is pressed between clamp jaws.

As can be seen in FIG. 7, the entrance to each blood flow channel is initially deep but uniformly decreases in depth, as the flow channels widen, such that the cross-sectional area of each is substantially maintained while the depth is decreased. This design enhances uniform flow in the module and allows the flow conditions in the thin channels to be attained more gradually than if the entrances to the channels were also thin. The initial depth is greater than about 10 mils (.3 mm), preferably about 15 to 20 mils (.4 to .5 mm) and is gradually decreased to about 4 to 10 mils (.1 to .3 mm).

Envelope 30 allows the module to be purged of air and filled with a liquid, e.g., saline, prior to use. When the module is used, this saline solution is swept out of the flow channels by blood and plasma but remains around the periphery of envelope 30 in the region of seal 31. Any blood which may leak into this solution in this region remains there by a check-valve action, due to the seal between perimeter channels 34' and the perimeter sealing boss 45 illustrated in FIGS. 5 and 6, similar to that described for the sealing region surrounding inlet 24 in FIG. 7.

As shown in FIG. 8, tube 48 connections to the apertures 20, 33, 29 are made by joining flanged plastic fittings 49 to the plastic envelope 30 on the bottom of the unit as seen in FIG. 4. No direct connection is made to any of the plates 19B, 19A, 32; however, the fittings are urged against the envelope 30 and into shallow counterbores 50 in end plate 19B by means of a clamping mechanism, namely, jaws 51, 52. Counterbores 50 prevent the plates from moving relative to the envelope during use. Jaws 51, 52, faced with elastomer 53, 53', engage envelope 30 at the top of plate 19A and the bottom of plate 19B and, in addition to holding the tube fittings, urge the stacked plates together in leak-tight condition resisting the hydrostatic pressure of the blood being pumped through the module. Unit pressures within the module are in the order of .5 to 3 psi (3.4 to 20.7 kPa) on an area of 40 sq in (250 sq. mm) resulting in clamp loadings of

120 lb (54.4 × 10³ gm). The clamp must provide sufficient external pressure to offset this internal pressure as well as to compensate for compliance and manufacturing tolerances. This external pressure should be evenly distributed.

Referring to FIG. 9, jaw 52 is a rectangular platen having yoke 54 bolted thereto. Yoke 53 has four legs, two shown; the number of legs is not critical. Jaw 51 is a floating and self-aligning circular platen of larger diameter than module 55 which is pressed against module 55 by means of central gear-reduced screw 56 extending through yoke 54 and connected to jaw 51 by means of a swivel joint, not shown. A gear-reducing mechanism, not shown, is fitted to the top of yoke 54. Two bosses 57, shown cut off, are on either side of screw 56 and house reciprocating plungers, as further described below with reference to FIG. 10. Elastomer 53, 53' lie between jaws 51, 52 and module 55. A guide pin, not shown, extending through yoke 54 to jaw 51 is used to properly align jaw 51 with module 55 upon clamping. It has been found that use of such a clamping mechanism provides nearly uniform pressure across the module and provides structural support external to the module, thereby lowering the cost of the module which is a disposable unit.

FIG. 10 illustrates the reciprocating plungers of a pulse generator integral with jaw 51. It is a cross section taken perpendicular to the cross section of FIG. 9. Jaw 51 bosses 57 for two parallel bores occupied by plungers 58 which are shouldered to carry springs 59 which urge the plungers toward reciprocatory pulse cavities. The plungers are lifted 180° out of phase with each other by means of eccentrics 60 on a common shaft 61 which is carried in bearings, not shown, and is extended outside the bar for a belt connection to a motor drive not shown, which is mounted on brackets, not shown, extending from jaw 51. The eccentrics 60 each engage a roller 62 in a slot in each plunger 58. Each roller 62 is carried on a wrist pin in the plungers. The throw of the eccentrics is about 0.030 inch (.8 mm) producing a plunger stroke of about 0.060 inch (1.6 mm). The eccentric shaft drives the pistons down away from the diaphragm compressing the springs and storing energy. The pistons are returned by the springs which limit the maximum force and resulting pressure which can be generated by the piston on the diaphragm over each cavity. This also limits jamming damage should the unit be installed misaligned or with a foreign body in the clamp cavity area.

The bottom of jaw 51 is pressed against plastic envelope 30 by the clamp so that the plunger heads 58 enter the reciprocatory pulse cavities. Rotation of shaft 61 causes diaphragm-like deflections in envelope 30 and produces a pumping action on fluids in the cavities. This action is oscillatory, causing reciprocatory pulsatile flow on the surfaces of the membrane filters. Because the reciprocatory pulse cavities are integral with the modular assembly of stacked plates and filter membranes, there is minimal addition to the average hold-up time of the blood being processed and each flow fraction receives uniform treatment.

FIGS. 11 and 12 illustrate, respectively, an alternative blood side support and an alternative plasma side support which may be used in a module of the invention. The supports and module are not the inventions claimed herein but are included because they represent embodiments which are useful with the invention herein. Referring to FIG. 11, at the center of the plate is blood flow channel inlet 63 surrounded by counterbore 64, which is about 0.5 inch (12.7 mm) in diameter and about 20 mils (.5 mm) in depth. From the counterbore, radial flow channels 65, shown enlarged and in part, are narrow around the inlet and extend to a perimeter plasma-depleted blood collection channel which is a series of plasma-depleted blood collection channels 66, 67, 68. These channels lead to plasma-depleted blood outlet 69. In the illustration is shown a first perimeter channel 66 which has four equidistant exits to intermediate channels 67 each of which in turn have an exit to final channel 68. Each channel is about .070 (1.8 mm) wide × .030 inch (0.8 mm) deep. These channels comprise blood pressure balancing and sealing grooves serving, in this regard, the same purpose as the perimeter border of short, narrow channels 34' in FIG. 5. The channels are spaced inward in region 70 to avoid plasma channels and ports.

FIG. 12 illustrates a plasma side support which may be used with the alternative blood side support of FIG. 11. It differs from the plasma side support described above in FIG. 6 in the locations of blood outlet 69 and plasma outlet 71, the latter of which is in a protrusion 72 from the edge of the plate in order to avoid the various blood flow channels and ports. Sealing around the inlet is accomplished as illustrated above in FIG. 7. Sealing around the perimeter is effected by a check-valve-like action resulting from pressing a membrane between channels 66, 67, 68 on the blood side support and perimeter sealing surface 73 on the plasma side support, in a manner similar, though not as effective, as described above with reference to FIG. 7.

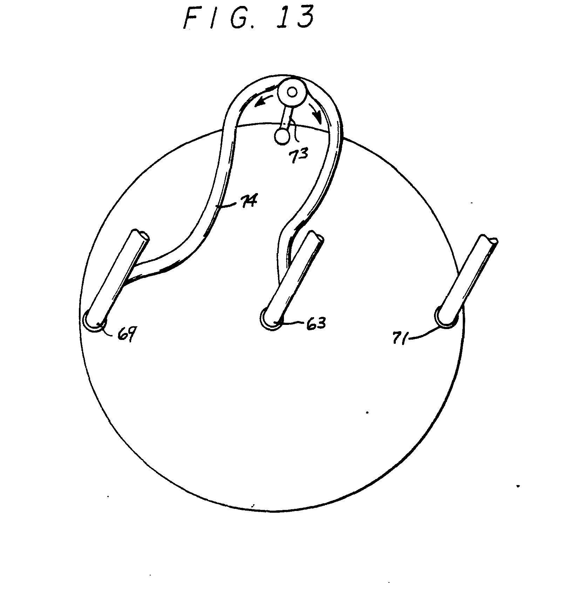

A second illustrative embodiment of a module, which can be used in the invention, employing the blood and plasma side supports of FIGS. 11 and 12, is shown in FIG. 13. This illustrative embodiment is not fitted with reciprocatory pulse cavities. Blood enters the module through blood flow path inlet 63, passes through blood flood flow channels, not shown, in blood side supports, not shown, and exits through plasma-depleted blood outlet 69. Plasma which passes through membranes between plasma and blood side supports flows radially to plasma collection channels and out of the module via plasma outlet 71. An external clamping mechanism, such as the mechanism described above, absent the pulse generator, is used to provide structural support.

Reciprocatory pulsations are generated by an oscillating peristaltic pump 73 on a loop 74 of flexible tubing extending between blood inlet 63 and plasma-depleted blood outlet 69. Pulse volume can be changed, e.g., by changing stroke length or the diameter of tubing used for the loop.

## Examples

In all of the following examples which are illustrative of treatments to separate plasma from blood in accordance with the invention, compatibility-matched human blood collected in either ACD or heparin was used. Unless otherwise noted, the hematocrit of the blood, maintained at 37°C during treatment, was 37—38%.

In Examples 1 to 6, planar circular supported membranes were encased in membrane filter modules made from Du Pont Lucite® acrylic resin. The membrane filter modules each comprised two circular discs between which were placed one or two supported membranes. Blood was fed to an inlet port at the center of the module and conducted radially therefrom across the surface of each membrane. Plasma-depleted blood and plasma were collected by means of peripheral channels, cut into the discs, which led to outlet ports.

The membranes were polycarbonate capillary pore membranes, available from Nuclepore Corporation, having average cell-retaining pore diameters of about 0.4 μm, about 10% pore area, a break elongation of 10 to 15%, machine direction, and 25 to 30%, cross direction, and a thickness of about 10 μm.

Three materials were alternatively used in the construction of membrane supports. One of these was Hollytex and two were high density polyethylene. Hollytex is a nonwoven polyester fabric produced from layers of Du Pont Reemay® spunbonded polyester by a calendering procedure. The Hollytex material was used in layers 10 mils (254 μm) or 4 mils (102 μm) thick. The polyethylene materials were porous plates about 6.3 mils (160.0 μm) thick; one had pores which were about 70 μm and the other, about 120 μm, in diameter. Radial channels in the disc below the polyethylene plate allowed for lateral flow of plasma.

Prior to each treatment, the module was purged of air by flushing with saline. The Hollytex supports were first solvent-exchanged in isopropanol, soaked in saline and then placed wet in the membrane filter module. The membrane filter module was submerged in saline, 37°C, during treatment to prevent air leakage. Removing air from and maintaining air out of the module is important.

The transmembrane pressure difference was measured by means of pressure strain gauge transducers and monitored near the center and/or near the periphery (outlet) of the module and was recorded, usually at 5 to 10 min intervals. The plasma side of the apparatus was vented, except where noted, and was assumed to be at atmospheric pressure.

Hemolysis was determined by visual observation of samples of plasma periodically collected during each treatment.

The operating conditions and results of each example are tabulated after a general description of the apparatus used therein. Elapsed time is in minutes and indicates the times during each treatment when measurements were taken. Peak and low pressures are in psig (kPa) and were measured near the indicated locations. Blood flow rate is the rate of flow of whole blood from the source to the module in mL per min. Plasma flow rate is the flow rate of collected plasma in mL per min. The hematocrit (Hct.) of plasma-depleted blood which was collected was calculated. Flux is mL of plasma collected per min per cm$^2$ of membrane filter.

## Example 1

This example illustrates plasmapheresis by reciprocatory pulsatile filtration using two membranes in a membrane filter module such that blood is filtered by both membranes simultaneously.

Two layers of Hollytex were placed between two membranes so that blood flowed across the first surfaces of both membranes within recessed flow regions cut into the inside surfaces of the discs and plasma which passed through the membranes flowed radially through the support between the membranes. The blood flow paths were about 8 mils (203.2 μm) in depth and had a combined surface area of about .05 m$^2$. Plasma-depleted blood from the ends of the flow paths was conducted further through outlet ports and collection tubing to a collection vessel.

Blood was conducted forward and reverse by two pumps which were similar to the hose pump described in U.K. Specification 2,020,735, published November 21, 1979, except that the outlet valves were removed, and which were positioned between the blood bag and the membrane filter module. Each pump comprised an inlet valve and a 4 inch (10.2 cm) plunger. The inlet valves were closed while the plungers were rising and were partially closed while the plungers were withdrawing so that blood was conducted from the directions of the blood bag and of the membrane filter module as the plungers were withdrawing. The plungers never completely occluded the tubing. Each pump displaced about 3.2 mL during each forward pulse.

Blood passed from the blood bag through a single tubing which was divided into two lines. Each line passed through one of the pumps and was rejoined into a single line.

Blood was also conducted in the reverse direction by pressure which accumulated in a surge chamber of about 50 mL which was connected by tubing to the blood flow path at two locations near the end of the flow path.

A .33 psi (2.3 kPa) check valve prevented backflow of blood to the blood bag. A control valve on the plasma-depleted blood collection line was adjusted during the treatment to control blood side pressures and transmembrane pressure difference.

The conditions and results of this example are in Table 1.

## TABLE 1

| Elapsed Time | Pulsations per min. (combined) | Blood Flow Rate | Peak Pressure Blood | | Low Pressure Blood | | Plasma Flow Rate | Hct. | Flux |
|---|---|---|---|---|---|---|---|---|---|
| | | | Inlet | Outlet | Inlet | Outlet | | | |
| 6.0 | 60 | 32.67 | 2.5(17.2) | 1.3(9.0) | -2.0(-13.8) | .4(2.8) | 10.61 | 56.2 | .0254 |
| 10.5 | 60 | 23.82 | 2.9(20.0) | 2.0(13.8) | -1.9(-13.1) | .8(5.5) | 8.88 | 60.6 | .0212 |
| 15.0 | 60 | 15.95 | 3.3(22.8) | 2.6(17.9) | -1.5(-10.3) | 1.2(8.3) | 6.26 | 62.5 | .0150 |
| 24.0 | 60 | 16.02 | 2.9(20.0) | 2.4(16.5) | -1.4(-.9.7) | 1.0(6.9) | 6.45 | 63.6 | .0154 |
| 27.5 | 65 | 17.43 | 3.1(21.4) | 2.5(17.2) | -1.3(-9.0) | 1.1(7.6) | 6.93 | 63.1 | .0166 |
| 33.5 | 65 | 13.29 | 3.5(24.1) | 2.9(20.0) | -1.1(-7.6) | 1.4(9.7) | 5.49 | 64.7 | .0131 |
| 39.5 | 75 | 13.82 | 3.8(26.2) | 3.3(22.8) | -.9(-6.2) | 1.6(11.0) | 5.62 | 64.0 | .0134 |
| 45.5 | 100 | 17.07 | 4.8(33.1) | 4.1(28.3) | -.1(-.7) | 2.2(15.2) | 6.62 | 62.0 | .0158 |
| 53.0 | 100 | 7.57 | 5.7(39.3) | 5.0(34.5) | .8(5.5) | 3.0(20.7) | 3.80 | 76.3 | .0091 |
| 59.5 | 60 | 20.54 | 4.4(30.3) | 3.7(25.5) | -.4(-2.8) | 1.8(12.4) | 6.62 | 56.0 | .0158 |

No hemolysis was observed during the first 39.5 minutes. Hemolysis was observed during the period when the frequency of pulsations was increased to 100 as a result, it is believed, of the high frequency and the high peak transmembrane pressure difference. After the pump speed and pressure were reduced, the plasma began to clear, indicating that hemolysis had ceased or was lessening.

Example 2

This example illustrates plasmapheresis by reciprocatory pulsatile filtration as per the invention using a single membrane.

The membrane was supported by a polyethylene plate (120 µm pores). The flow path surface area was about .013 m². The depth of the flow path was about 6 mils (152 µm) from the center thereof to a point along its radius about 3.25 inches (8.3 cm) therefrom, from which point, the depth tapered to about 9 mils (229 µm) at the end of the flow path. The peripheral edge of the membrane was pressed between the discs. Blood flowed radially across the first surface of the membrane while plasma which passed through the membrane passed through the pores in the polyethylene plate and flowed radially in a plasma flow region cut into the inside surface of the plasma-side disc.

Reciprocatory pulsatility and reductions in transmembrane pressure difference were provided by a peristaltic rotary pump which was modified by removal of all but one of the rollers therein. The circular path of the roller was about 5.38 inches (13.65 cm) of which the roller occluded the tubing for about 5.25 inches (13.34 cm); the tubing was .13 inch (.32 cm) ID silicon tubing. Therefore, the displacement of the pump, which was set at 60 rpm, was about 1.1 mL.

A check valve and plasma-depleted blood control valve were used.

The peak plasma side pressure remained at about 1.0 psi (6.9 kPa) at the center and periphery of the module; the low plasma side pressure was about 0 to 0.3 psi (0 to 2.1 kPa) at the center and periphery.

No hemolysis was observed. The conditions and results of this example are in Table 2.

TABLE 2

| Elapsed Time | Blood Flow Rate | Peak pressures Blood | | Low pressures Blood | | Plasma Flow Rate | Hct. | Flux |
|---|---|---|---|---|---|---|---|---|
| | | Inlet | Outlet | Inlet | Outlet | | | |
| 16 | 6.73 | 3.5(24.1) | 3.5(24.1) | .1(.7) | .4(2.8) | 1.98 | 52.4 | .0158 |
| 19 | 6.14 | 3.9(26.9) | 3.8(26.2) | .1(.7) | .6(4.1) | 2.59 | 63.9 | .0207 |
| 28 | 2.13 | 4.2(29.0) | 4.3(29.6) | .1(.7) | 1.0(6.9) | 1.01 | 70.3 | .0080 |
| 32 | 8.44 | 3.4(23.4) | 3.4(23.4) | 0(0) | .2(1.4) | 1.51 | 45.0 | .0120 |
| 38 | 4.24 | 3.8(26.2) | 3.9(26.9) | 0(0) | .5(3.5) | 1.77 | 63.5 | .0141 |
| 44 | 5.40 | 3.7(25.5) | – | 0(0) | – | 1.68 | 53.7 | .0134 |

Example 3

This example illustrates that reciprocatory pulsatile flow during plasmapheresis can result in an improved rate of plasma separation per unit area of membrane as compared to pulsatile flow without reciprocatory pulsations.

The membrane filter module was the same as in Example 2 except that the entire flow path depth was about 6 mils (152 µm) in depth and the porous plate had pores which were about 70 µm in diameter.

Initially, blood was conducted forward by a pressure infuser cuff wrapped around the blood bag. A 0.5 inch (1.3 cm) ID control valve positioned between the bag and the module was opened and closed at various intervals (reported in seconds) to generate pulsatile flow. After some time, the infuser cuff was removed and the rotary pump described above in Example 2 was utilized. Then the rotary pump was disconnected and the infuser cuff system was restored.

The conditions and results of this example are in Table 3.

## TABLE 3

| Elapsed Time | Pump | Blood Flow Rate | Peak Pressure | | Low Pressure | | Plasma Flow Rate | Hct. | Flux |
|---|---|---|---|---|---|---|---|---|---|
| | | | Blood Inlet | Plasma Outlet | Blood Inlet | Plasma Outlet | | | |
| 25 | infuser open: 30 closed: 15 | 7.65 | 3.8(26.2) | .1(.7) | .3(2.1) | -.3(-2.1) | .78 | 42.6 | .0062 |
| 36 | infuser open: 20 closed: 25 | 4.34 | 3.7(25.5) | .2(1.4) | .3(2.1) | -.3(-2.1) | .42 | 47.0 | .0033 |
| 41 | rotary | 10.40 | 4.3(29.6) | .1(.7) | .3(2.1) | -.9(-6.2) | 2.62 | 50.7 | .0209 |
| 45 | " | 6.20 | 4.2(29.0) | .4(2.8) | .4(2.8) | -.6(-4.1) | 2.20 | 58.9 | .0176 |
| 55 | infuser open: 4 closed: 12 | 3.65 | 3.4(23.4) | .4(2.8) | .6(4.1) | -.2(-1.4) | .40 | 42.6 | .0032 |
| 62 | " | 3.44 | 3.3(22.8) | – | .5(3.4) | – | .40 | 43.0 | .0032 |
| 69 | infuser open: 10 closed: 12 | 3.79 | 3.2(22.1) | – | .5(3.4) | – | .45 | 43.0 | .0036 |
| 74 | infuser open: 20 closed: 20 | 3.23 | 3.1(21.4) | – | .2(1.4) | – | .44 | 43.9 | .0035 |

Hemolysis was observed to result when pulsations were generated by the infuser cuff/valve system but not when reciprocatory pulsations were generated by the rotary pump.

## Example 4

In this example, modules substantially as illustrated in FIGS. 1 and 2 were employed. The membranes were 7 inches (178 mm) in diameter, and provided a total membrane surface area of about .05 m$^2$. The plasma side supports comprised three layers of 4 mil (102 µm) thick Hollytex. A peripheral plasma-depleted blood collection channel, about 3.2 mm wide and 1.6 mm deep, surrounded the blood flow region of each plate. The membranes were adhered to circular plates, made from Du Pont Lucite® acrylic resin, with General Electric RTV 102 silicon adhesive which had a break elongation of 400%, a tensile strength of 350 psi (2.4 MPa), and a Shore A hardness of 30. The adhesive was applied by hand in a layer about 3 mils (76 µm) thick.

The same adhesive was used to form blood side supports by placing dots of the adhesive, between the membranes in two concentric circles. The blood flow path between the membranes was about 8 mils (.20 mm) deep. The adhesive supports were cured on the blood side surface of one membrane at 60°C overnight prior to assembly of the module. The plates were held together with clamps, without O-rings.

The blood was conducted forward by a 3-arm rotary peristaltic pump. A .33 psi (2.3 × 10$^3$ Pa) check valve was located between this pump and the blood reservoir.

Reciprocatory pulsations and pressure fluctuations were provided by a modified peristaltic pump, positioned on a loop, i.e., a length of tubing, which extended from two peripherally located ports and one centrally located port. The pump was modified so that a single roller, in constant contact with the tubing, oscillated in about a 50 mm stroke at about 40 cycles-min$^{-1}$, thereby displacing about 1.6 mL per stroke. A micrometer control valve was placed on the plasma-depleted blood outlet line and was adjusted during the treatment.

Results and conditions of this treatment are summarized in Table 4. No hemolysis was observed.

TABLE 4

| Elapsed Time (min) | Blood Inlet Flow Rate ($mL\text{-}min.^{-1}$) | Blood Flow Path Pressure | | | | Plasma Flow Rate ($mL\text{-}min.^{-1}$) | Outlet Hct. | Flux |
|---|---|---|---|---|---|---|---|---|
| | | Peak | | Low | | | | |
| | | Inlet | Outlet | Inlet | Outlet | | | |
| 3.0 | 23.7 | 1.5(10.3) | .6(4.1) | -.8(5.5) | -.6(4.1) | 8.6 | 59.5 | .017 |
| 8.5 | 14.1 | 2.2(15.2) | 2.0(13.8) | -.4(2.8) | -.1(.7) | 8.2 | 90.1 | .016 |
| 12.5 | 15.7 | 3.0(20.7) | 3.0(20.7) | .5(3.4) | .3(2.1) | 8.8 | 86.3 | .018 |
| 17.0 | 15.4 | 3.0(20.7) | 2.9(20.0) | .8(5.5) | .6(4.1) | 8.2 | 81.1 | .016 |
| 22.0 | 14.6 | 2.7(18.6) | 2.8(19.3) | .6(4.1) | .7(4.8) | 8.4 | 88.9 | .017 |
| 25.5 | 15.1 | 3.6(24.8) | 3.7(25.5) | 1.9(13.1) | 1.8(12.4) | 8.5 | 86.6 | .017 |

Example 5

The apparatus used in this example was identical to that described above in Example 4 except that the module was smaller, the membranes being about 6 inches (152 mm) in diameter and providing a total membrane surface area of about .04 m².

Results and conditions of this treatment are summarized in Table 5. No hemolysis was observed.

TABLE 5

| Elapsed Time (min) | Blood Inlet Flow Rate $(mL\text{-}min.^{-1})$ | Blood Flow Path Pressure | | | | Plasma Flow Rate $(mL\text{-}min.^{-1})$ | Outlet Hct. | Flux |
|---|---|---|---|---|---|---|---|---|
| | | Peak | | Low | | | | |
| | | Inlet | Outlet | Inlet | Outlet | | | |
| 3.0 | 22.1 | 2.0(13.8) | .7(4.8) | −1.2(8.3) | −.8(5.5) | 9.4 | 66.2 | .024 |
| 7.0 | 23.4 | 3.0(20.7) | 2.0(13.8) | −.3(2.1) | 0(0) | 12.3 | 80.3 | .030 |
| 12.5 | 10.2 | 3.4(23.4) | 2.3(15.9) | −1.0(6.9) | −.2(1.4) | 5.7 | 86.6 | .014 |
| 17.0 | 25.4 | 3.3(22.8) | 2.5(17.2) | .3(2.1) | .5(3.4) | 13.0 | 77.9 | .032 |
| 20.5 | 25.2 | 4.4(30.3) | 3.9(26.9) | 1.9(13.1) | 2.2(15.2) | 12.8 | 76.2 | .032 |

### Example 6

The apparatus used in this example was substantially identical to that described in Example 5. The stroke length of the oscillating pump was varied during the treatment. The oscillator was turned off for a three minute interval so that, during this period, blood was being conducted forward only. The inlet hematocrit was 37%.

Results and conditions of this treatment are summarized in Table 6. Slight hemolysis was briefly observed when the stroke length was changed from four inches (101 mm) to three inches (76 mm) and again when the oscillation was turned on after the one minute interval of constant flow.

TABLE 6

| Elapsed Time (min) | Oscillator Stroke Length (mm) | Blood Inlet Flow Rate $(mL-min.^{-1})$ | Blood Flow Path Pressure | | | | Plasma Flow Rate $(mL-min.^{-1})$ | Outlet Hct. | Flux |
|---|---|---|---|---|---|---|---|---|---|
| | | | Peak | | Low | | | | |
| | | | Inlet | Outlet | Inlet | Outlet | | | |
| 1 | 101 | 51.3 | 3.2(22.1) | 1.2(8.3) | -- | -1.0(6.9) | 13.9 | 50.7 | .035 |
| 3.5 | 76 | 53.5 | 2.5(17.2) | .7(4.8) | -1.0(6.9) | -.4(2.8) | 14.7 | 51.0 | .037 |
| 7.0 | 76 | 53.8 | 2.9(20.0) | 1.8(12.4) | .1(.7) | .3(2.1) | 18.1 | 55.7 | .045 |
| 9.0 | 76 | 53.4 | 4.0(27.6) | 3.5(24.1) | 2.1(14.5) | 2.1(14.5) | 17.9 | 55.6 | .045 |
| 10.0 | 0 | 53.7 | 3.5(24.1) | 3.5(24.1) | 3.4(23.4) | 3.4(23.4) | 3.8 | 38.8 | .010 |
| 13.0 | 76 | 52.9 | 2.3(15.9) | 1.5(10.3) | .3(2.1) | .5(3.4) | 9.8 | 45.3 | .025 |
| 16.0 | 51 | 54.1 | 3.2(22.1) | 2.6(17.9) | 1.7(11.7) | 1.7(11.7) | 16.4 | 53.0 | .041 |

## Examples 7 and 8

The conditions and results of each example are tabulated. Pulse frequency is the frequency of reciprocatory pulsations in strokes per minute. Blood flow ratre is the rate at which blood was conducted into the filtration module in mL per minute. STMP is the mean system transmembrane pressure difference in mm Hg (kPa), i.e. [(mean blood inlet pressure + mean plasma-depleted blood outlet pressure) ÷ 2] − (mean plasma outlet pressure). Plasma flow rate is the rate at which plasma was collected in mL per minute. Hct is the hematocrit, i.e., volume percent of red blood cells, of the plasma-depleted blood which was collected.

## Example 7

Anticoagulated human blood, having a hematocrit of 32% and being maintained in a first reservoir at 37°C, was conducted into a plasmapheresis filtration module which is substantially depicted by FIGS. 7, 11, 12 and 13. The module comprised six polycarbonate membranes, 10 μm thick, from Nuclepore Corporation, having 0.4 μm diameter pores. The total effective membrane surface area was 0.108 m². The height of the blood flow channels was 5 mils (.13 mm). The module did not comprise a sealing envelope. The plates were clamped and then sealed around the perimeter with vacuum grease.

Reciprocatory pulsations were generated by a peristaltic pump on a loop comprised of two lengths of flexible tubing joining an inlet and an outlet. The pump was modified to oscillate with a 53 mm stroke length and a displacement of 7.5 mL.

Pressure transducers were located on the blood inlet line, the plasma line and the plasma-depleted blood outlet line. Plasma flowed into a vented level control chamber and was conducted from the chamber by a metering pump. Plasma and plasma-depleted blood were collected in a second reservoir and recycled to the first reservoir.

Table 7 summarizes this example.

### TABLE 7

| Time (min.) | Blood Flow Rate | Pulse Frequency | STMP | | Plasma Flow Rate | · Hct. |
|---|---|---|---|---|---|---|
| 5.0 | 53 | 30 | 14 | (1.9) | 25.8 | 72 |
| 9.5 | 58 | 60 | 20 | (2.7) | 21.2 | 80 |
| 13.5 | 76 | 30 | 30 | (4.0) | 28.5 | 59 |
| 17.0 | 80 | 60 | 30 | (4.0) | 37.6 | 60 |
| 20.5 | 100 | 30 | 32 | (4.3) | 32.5 | 47 |
| 24.5 | 105 | 60 | 40 | (5.3) | 39.5 | 51 |
| 32.0 | 135 | 30 | 44 | (5.9) | 30.7 | 41 |
| 36.5 | 135 | 60 | 53 | (7.1) | 36.8 | 44 |
| 40.0 | 170 | 30 | 53 | (7.1) | 32.1 | 39 |
| 43.0 | 170 | 60 | 63 | (8.4) | 37.6 | 41 |
| 46.0 | 200 | 30 | 61 | (8.1) | 30.1 | 38 |
| 50.5 | 200 | 60 | 74 | (9.9) | 36.6 | 39 |

This example was continued for a total of 92 minutes. Results from 50.5 to 92 minutes are not reported due to intermittent failure of the oscillator during this time period.

## Example 8

Anticoagulated human blood, having a hemotocrit of 38% and being maintained in a reservoir at 37°C, was conducted into a filtration module which is substantially depicted by FIGS. 7, 11, 12 and 13. The module comprised a single polycarbonate membrane, 10 μm thick, from Nuclepore Corporation, having 0.6 μm diameter pores. The effective membrane surface area was 0.018 m². The height of the blood flow channels was 4.5 mils (.11 mm). The module did not comprise a sealing envelope. The plates were clamped and then sealed around the perimeter with vacuum grease.

Reciprocatory pulsations were generated in similar fashion to Example 7. The stroke length was 53 mm and the displacement was 0.42 mL.

Pressure transducers were located on the blood inlet line and the plasma-depleted blood outlet line. Plasma pressure was assumed to be atmospheric. Plasma and plasma-depleted blood were not recycled.

Table 8 summarizes this example.

## TABLE 8

| Blood Flow Rate | Pulse Frequency | STMP | Plasma Flow Rate | Hct. |
|---|---|---|---|---|
| 9.5 | 42 | 62 (8.3) | 4.5 | 72 |
| 10.6 | 42 | 98 (13.1) | 4.9 | 70 |
| 20.0 | 42 | 16 (2.1) | 3.5 | 46 |
| 20.4 | 42 | 70 (9.3) | 7.0 | 58 |
| 9.4 | 42 | 67 (8.9) | 4.4 | 71 |
| 9.1 | 20 | 70 (9.3) | 3.0 | 57 |
| 9.9 | 60 | 54 (7.2) | 4.8 | 74 |

Conditions and results are reported at approximately 4 to 6 minute intervals.

## Claims

1. Reciprocatory pulsatile flow method for continuously separating plasma from blood, which method comprises:

a) conducting blood in a forward direction over a first surface of each of one or more membranes having cell-retaining pores, while maintaining a net positive transmembrane pressure difference;

b) terminating the forward conducting of blood over the first surface of the membrane;

c) conducting the blood in the reverse direction over said first surface, the volume of blood flowed in the reverse direction being less than the volume of blood flowed in the forward direction in step a);

d) repeating steps a)—c) in sequence to superimpose oscillatory pulsatile flow on the continuous forward flow of the blood, and collecting plasma which passes through each membrane from a second surface thereof and collecting plasma-depleted blood from said first surface, with the proviso that said plasma and said plasma-depleted blood are not returned to the donor body from which the blood is drawn.

2. Method according to Claim 1 wherein reciprocatory pulsations are imparted to the blood in a blood flow path on said first surface of said membrane by oscillating the blood between a inlet and an outlet of the blood flow path.

3. Reciprocatory pulsatile flow apparatus for continuously separating plasma from blood, which apparatus comprises one or more membranes (10A, 10B; 47) having cell-retaining pores, means (15; 22, 27, 30, 58; 51—62; 73) for conducting blood forward at a net positive transmembrane pressure difference and reverse over a first surface of each membrane whereby to superimpose oscillatory pulsatile flow on the continuous forward flow of the blood, means (3A, 3B, 4A, 4B; 33; 41, 44; 71) for collecting plasma which passes through each membrane from a second surface thereof and means (7, 8; 28, 29; 34, 34′, 35, 25; 66—69) for collecting plasma-depleted blood from said first surface.

4. Apparatus according to Claim 3 having a blood flow path (2; 34; 65) on said first surface of said membrane, including means (15; 22, 27, 30, 58; 51—62; 73) for oscillating blood between an inlet (5; 24; 63) and an outlet (8; 25; 69) of the blood flow path.

## Patentansprüche

1. Vorwärts-Rückwärts-Strömungsverfahren zur kontinuierlichen Abtrennung von Plasma aus Blut, umfassend;

a) das Leiten von Blut in Richtung nach vorn über eine erste Oberfläche einer oder jeder von mehreren Membranen mit Zellen zurückhaltenden Poren, wobei insgesamt eine positive Druckdifferenz über der Membran aufrechterhalten wird;

b) das Beenden des Leitens von Blut nach vorn über die erste Oberfläche der Membran;

c) das Leiten des Blutes in der umgekehrten Richtung nach rückwärts über die genannte erste Oberfläche, wobei das Volumen des in rückwärtiger Richtung fließenden Blutes kleiner ist als das des in Schritt a) vorwärts fließenden Blutes;

d) das Wiederholen der Schritte a) bis c) nacheinander, um die kontinuierliche Vorwärts-Strömung des Blutes mit einer oszillierenden, pulsierenden Strömung zu überlagern, und

das Sammeln des Plasmas, das durch jede Membran hindurchtritt, von einer zweiten Oberfläche derselben und das Sammeln des an Plasma verarmten Blutes von der ersten Oberfläche, mit der Maßgabe, daß das Plasma und das an Plasma verarmte Blut nicht zu dem Spender-Körper zurückgeleitet werden, dem das Blut entnommen wurde.

2. Verfahren nach Anspruch 1, worin das Blut in einem Blutströmungsweg auf der ersten Oberfläche der Membran durch Oszilieren des Blutes zwischen einem Einlaß und einem Auslaß des BLutströmungsweges in pulsierende Hin- und Herbewegungen versetzt wird.

3. Vorwärts-Rückwärts-Strömungs-Apparat zur kontinuierlichen Abtrennung von Plasma aus Blut, umfassend eine oder mehrere Membranen (10A, 10B; 47) mit Zellen zurückhaltenden Poren, Mittel (15; 22, 27, 30, 58; 51—62; 73) zum Vorwärts-Leiten des Blutes bei einer insgesamt positiven Druckdifferenz über die Membran und zum Rückwärts-Leiten über eine erste Oberfläche jeder Membran, um die kontinuierliche Vorwärts-Strömung des Blutes mit einer oszillierenden, pulsierenden Strömung zu überlagern, Mittel (3A, 3B, 4A, 4B; 33; 33, 41, 44; 71) zum Sammeln des Plasmas, das durch jede Membran hindurchtritt, von einer zweiten Oberfläche derselben und Mittel (7, 8; 28;, 29; 34, 34', 35, 25; 66—69) zum Sammeln des an Plasma verarmten Blutes von der genannten ersten Oberfläche.

4. Apparat nach Anspruch 3, mit einem Blutströmungsweg (2; 34; 65) auf der ersten Oberfläche der Membran, umfassend Mittel (15; 22, 27, 30, 58; 51—62; 73) zum Oszillieren des Blutes zwischen einem Einlaß (5; 24; 63) und einem Auslaß (8; 25; 69) des Blutströmunsgeweges.

## Revendications

1. Procédé d'écoulement pulsatif réciproque pour séparer en continu du plasma du sang, méthode qui consiste à:

a) faire passer du sang dans un sens avant sur une première surface de chacune d'une ou plusieurs membranes ayant des pores de retenue de cellules, tout en maintenant une différence de pression nette positive à travers le membrane;

b) arrêter le passage avant du sant sur la première surface de la membrane;

c) faire passer le sang dans le sens inverse sur ladite première surface, le volume de sant écoulé dans le sens inverse étant inférieur au volume de sang écoulé dans le sens avant dals l'étape a);

d) répéter les étapes a)—c) en séquence pour superposer un écoulement pulsatif oscillatoire sur l'écoulement avant continu du sant, et recueillir le plasma qui passe à travers chaque membrane à partir d'une seconde surface de celle-ci et recueillir le sang appauvri en plasma à partir de ladite première surface, avec la condition que ledit plasma et ledit sang appauvri en plasma ne sont pas retournés au corps du donneur dont le sang est retiré.

2. Procédé selon la revendication 1 dans lequel des pulsations réciproques sont communiquées au sang dans un chemin d'écoulement du sang sur ladite première surface de ladite membrane en faisant osciller le sang entre une entrée et une sortie du chemin d'écoulement du sang.

3. Appareil d'écoulement pulsatif réciproque pour séparer en continu du plasma du sang, cet appareil comprenant une ou plusieurs membranes (10A, 10B; 47) ayant des pores de retenue de cellules, des moyens (15; 22, 27, 30, 58; 51—62; 73) pour faire passer le sang dans le sens avant avec une différence de pression nette positive à travers la membrane et dans le sens inverse sur une première surface de chaque membrane pour superposer un écoulement pulsatif oscillatoire sur l'écoulement avant continu du sang, des moyens (3A, 3B, 4A, 4B; 33; 33, 41, 44; 71) pour recueillir le plasma qui passe à travers chaque membrane à partir d'une deuxième surface de celle-ci et des moyens (7, 8; 28, 29; 34, 34', 35, w5'; 66—69) pour recueillir le sang appauvri en plasma à partir de ladite première surface.

4. Appareil selon la revendication 3 ayant un chemin d'écoulement (2; 34; 65) sur ladite première surface de ladite membrane, comprenant des moyens (15; 22, 27, 30, 58; 51—62; 73) pour faire osciller le sang entre une entrée (5; 24; 63) et une sortie (8; 25; 69) du chemin d'écoulement du sang.

F I G. 1

F I G. 2

FIG. 3

FIG. 4

FIG. 7

BLOOD

PLASMA

EP 0 070 738 B1

F I G. 5

# F I G . 6

# F I G. 8

# F I G. 10

6

# FIG. 9

F I G. 11

F I G. 12

*F I G. 13*